# EUROPEAN PATENT SPECIFICATION

(11) **EP 2 954 322 B1**
(45) Date of publication and mention of the grant of the patent: **18.07.2018**
(21) Application number: 14704321.0
(22) Date of filing: 10.02.2014
(51) Int. Cl.: G01N 33/50, C12N 5/077

(54) **IN VITRO METHOD FOR CARDIOVASCULAR RISK STRATIFICATION**
IN-VITRO-VERFAHREN ZUR STRATIFIZIERUNG DES KARDIOVASKULÄREN RISIKOS
PROCÉDÉ IN VITRO DE STRATIFICATION DU RISQUE CARDIOVASCULAIRE

(30) Priority: 08.02.2013 US 201361762468 P
(43) Date of publication of application: 16.12.2015
(73) Proprietor: Backs, Johannes, 69221 Dossenheim (DE); Katus, Hugo, 69120 Heidelberg (DE); Lehmann, Lorenz, 69115 Heidelberg (DE); Baysal-Temel, Ezgi, 20535 Hamburg (DE)
(72) Inventor: BACKS, Johannes, 69221 Dossenheim (DE); OEING, Christian, 69120 Heidelberg (DE); VOLZ, Christian, 69123 Heidelberg (DE); KATUS, Hugo, 69120 Heidelberg (DE); HERZIG, Stephan, 69251 Gaiberg (DE); BAYSAL, Ezgi, 22765 Hamburg (DE); SCHÄFER, Michaela, 69124 Heidelberg (DE); LEHMANN, Lorenz, 69115 Heidelberg (DE)
(74) Representative: Grahn, Sibylla Maria
(86) International application number: PCT/EP2014/052535
(87) International publication number: WO 2014/122302

(56) References cited:
- WO-A1-2012/055828
- TANAKA T ET AL: "In vitro pharmacologic testing using human induced pluripotent stem cell-derived cardiomyocytes", BIOCHEMICAL AND BIOPHYSICAL RESEARCH COMMUNICATIONS, ACADEMIC PRESS INC. ORLANDO, FL, US, vol. 385, no. 4, 7 August 2009 (2009-08-07), pages 497-502, XP026211098, ISSN: 0006-291X, DOI: 10.1016/J.BBRC.2009.05.073 [retrieved on 2009-05-21]
- ASAI YASUYUKI ET AL: "Combination of functional cardiomyocytes derived from human stem cells and a highly-efficient microelectrode array system: an ideal hybrid model assay for drug development", CURRENT STEM CELL RESEARCH & THERAPY, SAIF ZONE, SHARJAH [U.A.] : BENTHAM, AE, vol. 5, no. 3, 1 September 2010 (2010-09-01), pages 227-232, XP009155001, ISSN: 1574-888X
- GÃ BOR FÃLDES ET AL: "Modulation of human embryonic stem cell-derived cardiomyocyte growth: A testbed for studying human cardiac hypertrophy?", JOURNAL OF MOLECULAR AND CELLULAR CARDIOLOGY, ACADEMIC PRESS, GB, vol. 50, no. 2, 26 October 2010 (2010-10-26), pages 367-376, XP028132046, ISSN: 0022-2828, DOI: 10.1016/J.YJMCC.2010.10.029 [retrieved on 2010-11-01]
- ZAKYNTHINOS E ET AL: "Diagnostic and prognostic impact of brain natriuretic peptide in cardiac and noncardiac diseases", HEART AND LUNG, MOSBY, ST. LOUIS, US, vol. 37, no. 4, 1 July 2008 (2008-07-01), pages 275-285, XP022831488, ISSN: 0147-9563, DOI: 10.1016/J.HRTLNG.2007.05.010 [retrieved on 2008-07-10]
- W. A. LAFRAMBOISE ET AL: "Cardiac fibroblasts influence cardiomyocyte phenotype in vitro", AJP: CELL PHYSIOLOGY, vol. 292, no. 5, 1 May 2007 (2007-05-01), pages C1799-C1808, XP055112761, ISSN: 0363-6143, DOI: 10.1152/ajpcell.00166.2006
- PIPPA F. COSPER ET AL: "Interferon-[gamma] Causes Cardiac Myocyte Atrophy via Selective Degradation of Myosin Heavy Chain in a Model of Chronic Myocarditis", THE AMERICAN JOURNAL OF PATHOLOGY, vol. 181, no. 6, 1 December 2012 (2012-12-01), pages 2038-2046, XP055112727, ISSN: 0002-9440, DOI: 10.1016/j.ajpath.2012.08.040
- ASHLEY WYSONG ET AL: "NF-[kappa]B Inhibition Protects against Tumor-Induced Cardiac Atrophy in Vivo", THE AMERICAN JOURNAL OF PATHOLOGY, vol. 178, no. 3, 1 March 2011 (2011-03-01) , pages 1059-1068, XP055112737, ISSN: 0002-9440, DOI: 10.1016/j.ajpath.2010.12.009
- Belury: "Cardiac alterations in cancer-induced cachexia in mice", International journal of oncology, vol. 37, no. 2, 23 June 2010 (2010-06-23), XP055316274, GR ISSN: 1019-6439, DOI: 10.3892/ijo_00000683
- Martha Belury: "Evidence for cardiac atrophic remodeling in cancer-induced cachexia in mice", International journal of oncology, 5 August 2011 (2011-08-05), XP055316279, GR ISSN: 1019-6439, DOI: 10.3892/ijo.2011.1150

## Description

The present invention relates to an *in vitro* method for assessing the risk of developing a cardiovascular condition in a patient and/or for establishing a cardiovascular risk profile of a patient. It further relates to a kit for use in such method.

Cardiovascular diseases are the most common cause of death. They can develop on the basis of co-morbidities like diabetes, metabolic syndromes, obesity, arterial hypertension, chronic kidney disease, lipid storage diseases, chronic inflammation, environmental stress, depression, cancer, and many other factors. One such co-morbidity is tumor-induced cachexia, which is a complex metabolic syndrome characterized by atrophy of muscle and fat tissue with different extent in each patient even depending on the type of tumor disease. The mechanisms of this syndrome with its circulating metabolites remain mostly unclear. In particular the cardiac manifestation of this systemic reaction is subject to current investigations.

At present, cardiac dysfunctions can be diagnosed via imaging techniques like echocardiography or cardiovascular magnetic resonance (CMR). Laboratory tests like the measurement of NT-proBNP or functional tests like ergospirometry permit to assess the risk of an already diagnosed cardiac disease. However, a prediction as to whether or not a person suffering from a condition, such as tumor-induced cachexia, diabetes or depression, will actually develop a cardiac dysfunction has so far been very limited. Prediction was mostly based on the presence or absence of cardiovascular risk factors like smoking, positive family history, hypertension, alcohol abuse or present co-morbidities. An individual risk prediction has not been possible so far.

WO 2012/055828 A1 discloses a method of determining the risk of drug induced arrhythmia using stem cell derived cardiomyocytes in a high-throughput impedance or multi-electrode array assay.
Tanaka *et al*. (2009) disclose *in vitro* pharmacologic testing using human induced pluripotent stem-cell derived cardiomyocytes.

Asai *et al*. (2010) disclose the combination of functional cardiomyocytes derived from human stem cells and a microelectrode array system as an hybrid model assay for drug development. Tian *et al*. (2010) describe cardiac alterations in cancer-induced cachexia in mice. Tian *et al*. (2011) describe evidence for cardiac atrophic remodelling in cancer-induced cachexia in mice.

Accordingly, it was an object of the present invention to provide a straight-forward and efficient *in vitro* method for assessing the individual risk of developing a cardiovascular condition in a patient and/or for establishing an individual cardiovascular risk profile of a patient.

The object of the present invention is solved as claimed in the claims.

The object of the present invention is solved by an *in vitro* method for assessing the risk of developing a cardiovascular condition in a patient, said method comprising the steps:
- exposing a first group of cardiomyocytes to a sample derived from said patient;
- measuring at least one property of said cardiomyocytes;
- comparing said at least one property with the same at least one property measured in a second group of said cardiomyocytes, wherein said second group of said cardiomyocytes has been exposed to a control sample;
wherein a difference in said at least one property between said first group and said second group indicates an increased risk of developing said cardiovascular condition.

The patient is selected from the group of tumor patients, patients with tumor-induced cachexia, and patients suffering from depressive or other mood disorders.

In one embodiment, said cardiomyocytes are isolated from a non-human animal or said cardiomyocytes are derived from human or non-human induced pluripotent stem cells (iPSC), preferably human iPSC.

According to the invention, said sample is blood serum or blood plasma, preferably blood serum.

Preferably, the sample is diluted, e.g. with cell culture medium or another physiologically acceptable solution, such as PBS. Preferably, the sample is diluted to a concentration of from 0.1 to 20 % (v/v), preferably 0.1 to 7.5 % (v/v), more preferably 0.5 to 2.0 % (v/v).

Cell culture media for use with cardiomyocytes are known to a skilled person and include DMEM medium, medium 199, Ham's F10 and F12 media, MEM medium and mixtures thereof, e.g. DMEM/F12 or DMEM/199 medium. The media can further be supplemented, e.g. with growth factors and/or antibiotics. Appropriate supplements and their working concentrations are known to a person skilled in the art. Preferably, FCS, if present, is used at a concentration of 5 to 15%, preferably 10%.

The period of time, during which the cardiomyocytes are exposed to said sample derived from said patient (or said control sample(s)), may depend on the selection of said at least one property, i.e. the readout type. In one embodiment, the cardiomyocytes are exposed to said sample derived from said patient (or said control sample(s)) for 1 min to 3 days, preferably for 1 hr to 3 days, more preferably for 12 hrs to 3 days, even more preferably for 1 day to 3 days (24 to 72 hrs).

According to the invention, said at least one property is selected from the group comprising cell size, cell morphology, rate of apoptosis, expression of one or more genes or reporter constructs, presence of one or more nucleic acid molecules, epigenetic profile, biochemical parameters and cell function.

Preferably, the expression of gene(s) that are causative for heart failure, such as SERCA, S100A1, Myl12b and isotype switch of alpha to beta myosin heavy chain (MHC), or the expression of gene(s) that are associated with heart failure, such as ANP, BNP, osteopontin and ANGPTL7, can be measured and compared.

Preferably, biochemical parameters, such as post-translational modifications (PTMs), can be measured and compared, in particular the activation of CaMKII, phosphorylation and proteolysis of histone deacetylases, phosphorylation of phospholamban, troponins or Akt.

In one embodiment, said cardiovascular condition is selected from the group comprising cardiac atrophy, cardiac hypertrophy, heart failure and hereditary or acquired cardiomyopathies.

Cardiovascular risk factors include smoking, positive family history, hypertension, obesity, hypercholesterolemia, hypertriglyceridemia, alcohol abuse, lack of exercise, consumption of non-mediterranean food or present co-morbidities, such as diabetes, depression, cancer, inflammatory/autoimmune diseases and other systemic diseases.

A person skilled in the art knows how to choose a suitable control sample for a given sample derived from said patient and/or for a given cardiovascular condition and/or for a given at least one property to be measured. Preferably, said control sample is of the same type as the sample derived from said patient, i.e. when said sample derived from said patient is blood serum, said control sample is blood serum as well. Preferably, when said sample derived from said patient is diluted, the same dilution is used for said control sample.

In one embodiment, said control sample is a sample derived from a healthy individual or a mixture of samples derived from at least 3, preferably at least 5, more preferably at least 10 healthy individuals.

As used herein, the term "healthy individual" is meant to refer to a human individual between 18 and 50 years old who does not have said cardiovascular condition. Preferably, said human individual does not have any known cardiac diseases, cardiac symptoms like dyspnoea, angina pectoris, cardiovascular risk factors, known systemic diseases, known metabolic diseases, known endocrine disorder, known organ dysfunction, known abnormal habits (e.g. eating disorder, excessive exercise, immobility) and/or known mental illness.

In one embodiment, said at least one property is cell size, wherein, preferably,
said difference is a decreased cell size in said first group of cardiomyocytes and said cardiovascular condition is cardiac atrophy, or
said difference is an increased cell size in said first group of cardiomyocytes and said cardiovascular condition is cardiac hypertrophy.

In one embodiment, said *in vitro* method further comprises the step:
- comparing said at least one property with the same at least one property measured in a third group of said cardiomyocytes, wherein said third group of said cardiomyocytes has been exposed to a positive control sample for said cardiovascular condition.

A person skilled in the art knows how to choose a suitable positive control sample for a given cardiovascular condition.

In one embodiment, said cardiovascular condition is cardiac atrophy and said positive control sample is conditioned cell culture medium of colon-26 cells. Preferably, said conditioned cell culture medium is diluted.

In one embodiment, said cardiovascular condition is cardiac hypertrophy and said positive control sample is unconditioned cell culture medium supplemented with fetal calf serum (FCS) and, optionally, with phenylephrine, endothelin-1, prostaglandin E1, prostaglandin E2 and/or isoproterenol. Preferably, FCS is used at a concentration of 5 to 15%, preferably 10%.

A positive control sample for apoptosis is cell culture medium supplemented with doxorubicin. Preferably, doxorubicin is used at a concentration of 0.1 to 1.0 µM.

In one embodiment, said cardiomyocytes are provided in a 3D-culture system, for example an engineered heart tissue, as described, e.g. in Hirt M.N. et al., Basic Res. Cardiol., 107(6):307, 2012, or in Zimmermann W.H. et al., Biotechnol. Bioeng., 68(1):106, 2000.

In one embodiment, more than one property of said cardiomyocytes is measured.

In one embodiment, said step of measuring at least one property of said cardiomyocytes is performed with a plurality of properties of said cardiomyocytes, thereby establishing a cardiovascular risk profile of said patient.

The object of the present invention is also solved by a kit comprising
- a first container comprising cardiomyocytes, preferably cardiomyocytes derived from human or non-human induced pluripotent stem cells (iPSC), preferably human iPSC;
- a second container comprising a control sample which is a mixture of samples derived from at least 3, preferably at least 5, more preferably at least 10 healthy individuals; and
- optionally, at least one third container comprising a positive control sample for a cardiovascular condition as defined above.

The object of the present invention is further solved by the use of a kit as defined above for assessing the risk of developing a cardiovascular condition in a patient and/or for establishing a cardiovascular risk profile of said patient,
wherein said patient is selected from the group of tumor patients, patients with tumor-induced cachexia and patients suffering from depressive or other mood disorders.

The present inventors have developed a straight-forward *in vitro* test that can assess the individual cardiovascular risk of a patient via a simple measurement using a sample derived from the patient, e.g. blood serum. The method of the present invention can be automated and can potentially deliver predictive results for any possible combination and constellation of risk factors. The method can even be performed in a healthy population when individual risks are not immediately apparent. The method according to the present invention could be integrated into primary prevention diagnostics, as it provides the possibility to selectively and early detect patients that are prone to develop a cardiac disease and to preventatively treat them, for example, with typical heart failure therapy (beta-blockers, ACE inhibitors, mineralocorticoid antagonists etc.).

The *in vitro* method according to the present invention represents a novel possibility to predict an individual cardiac/cardiovascular risk. It is not based on the measurement of single selective factors, but on the effect of all metabolic factors contained in a sample derived from the patient, e.g. the patient's blood serum, on the cardiomyocytes' size, structure, transcription factor activities, gene expression, biochemical properties, expression of a reporter construct, genome, epigenome, activity und function. The identity of the single factors in the serum does not need to be known. Instead, the combined effects of all factors in the serum are tested.

The assay in accordance with the present invention can be performed with cells isolated from non-human animals, e.g. neonatal rat ventricular cardiomyocytes. Another possibility is the use of cardiomyocytes derived from non-human or human induced pluripotent stem cells (iPSCs). In this system, standardization and automation can be improved by producing large badges of iPSC-derived cardiomyocytes at once. For the assay, iPSC-derived cardiomyocytes will be thawed and plated, e.g. on 384-well plates, to then test the effects of the individual blood sera on, e.g., cell size or structure as a marker for remodelling (CardioS, "size of cardiomyocytes"). For example, an increase in size would be indicative for the development of heart failure, whereas a decrease in size (atrophy) would indicate the development of heart atrophy, another reason for heart failure.

The system can be further expanded by generating iPSCs that carry a luciferase, lacZ, RFP, GFP or other luminescent or fluorescent reporters for the activity of specific transcription factors, such as MEF2, SRF and NFAT (CardioR, "reporter activity of cardiomyocytes"). MEF2, SRF and NFAT activity is a specific marker of pathological remodeling in the heart and heart failure. A reporter assay can easily be automated.

Furthermore, epigenetic alterations as a measure for environment-gene interactions could be detected (CardioE, "epigenetics of cardiomyocytes"). One epigenetic marker that was recently identified is DNA methylation of the gene Myl12b. However, there are other epigenetic markers that could be used in accordance with the present invention, such as ANP, BNP, SERCA, Ly75, ADORA2, LMNA, MYH7 or BAG3 (some known from families with genetic cardiomyopathies having mutations in these genes). Possibly, a further extension can be performed to detect genome-wide changes of methylation via bisulphite sequencing or sequencing of enriched methylated genomic regions or sequencing of genomic regions associated to histone modifications (histone acetylation or methylation). The *ex vivo*/*in vitro* method of the present invention can help to avoid biopsies of heart tissue, as effects of blood sera on the epigenetics of cardiomyocytes can be measured directly.

Additionally, gene expression within the cardiomyocytes can be measured ("CardioGE", gene expression in cardiomyocytes), in particular the expression of genes that are causative for heart failure, such as SERCA, S100A1, Myl12b and isotype switch of alpha to beta myosin heavy chain (MHC), or the expression of genes that are associated with heart failure, such as ANP, BNP, osteopontin and ANGPTL7.

Moreover, biochemical parameters, such as biochemical post-translational modifications (PTMs), can be measured ("CardioB"), in particular the activation of CaMKII, phosphorylation and proteolysis of histone deacetylases, phosphorylation of phospholamban, troponins or Akt.

Lastly, the effects of blood sera on the function (e.g. contractility) of cardiomyocytes (CardioF, "function of cardiomyocytes") can be tested. For this approach, an automated "engineered heart tissue" (EHT) system can be used, wherein iPSC-derived cardiomyocytes together with cardiac fibroblasts are provided in a 3D culture (e.g. in a specific matrix gel) allowing to measure the contractility of the cardiomyocytes.

The above described CardioS, CardioR, CardioE, CardioGE, CardioB as well as CardioF, alone or in combination, can all be used to predict the cardiac risk in individual patients.

The *in vitro* method in accordance with the present invention is not limited to sera of tumor patients but can, e.g., be used in every systemic disease where one can think of secretion of metabolic factors as a contributing factor to progression of disease with cardiac involvement. Using the method of the present invention, chronic and systemic diseases may be categorized with regard to their cardiac risk profile. Potential patient groups include patients with cardiovascular risk factors, autoimmune diseases, endocrine syndromes, chronic inflammatory diseases that are potentially accompanied by cardiac involvement in the long run, but also patients with depressive disorders who exhibit a higher incidence of cardiovascular diseases and patients with other cardiovascular risk factors.

Patients whose samples, e.g. blood sera, show, for example, excessive atrophy or hypertrophy in the assay of the present invention could be subsequently examined via echocardiography or cardiac magnetic resonance (CMR). Follow-up examinations using established methods of cardiac patient care can further optimize the significance of the *in vitro* system according to the present invention.

### FIGURES

Reference is now made to the figures, wherein:

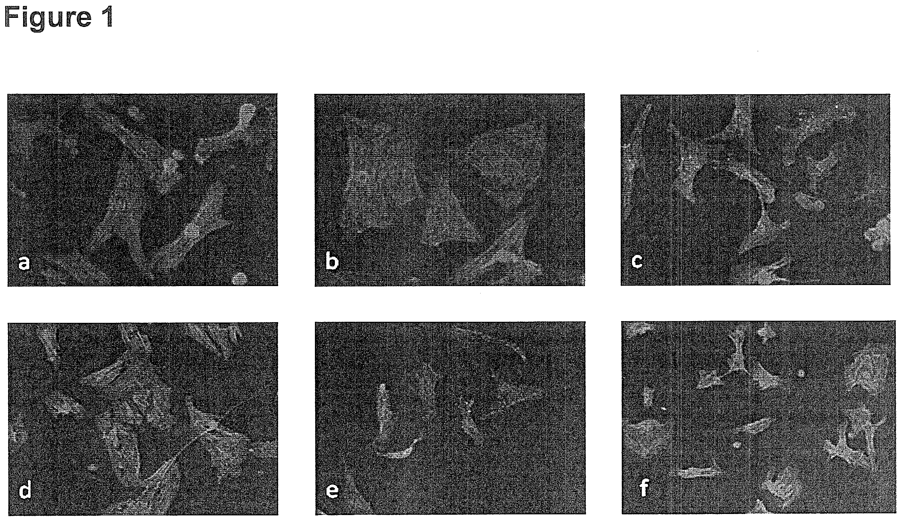

**Figure 1** shows the morphology of cardiomyocytes co-cultured with colon-26 tumor cells: a) serum-free without tumor cells; b) full medium without tumor cells; c) serum-free with tumor cells (low density); d) full medium with tumor cells (low density); e) serum-free with tumor cells (high density); f) full medium with tumor cells (high density).

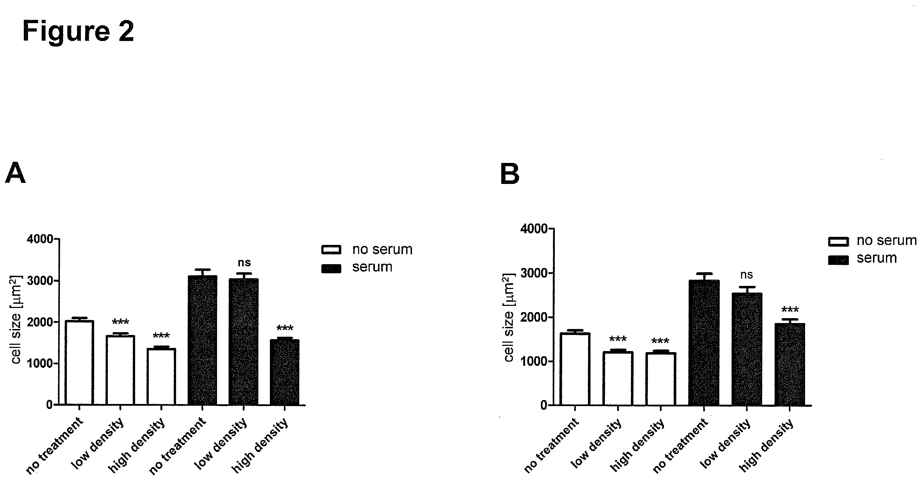

**Figure 2** shows the cell size of cardiomyocytes co-cultured with colon-26 tumor cells: A) preliminary and B) confirmatory experiment showing high reproducibility and marked atrophy in cardiomyocytes co-cultured with colon-26 tumor cells. Significance: *p < 0,05; **p < 0,01; ***p < 0,001, ns = not significant.

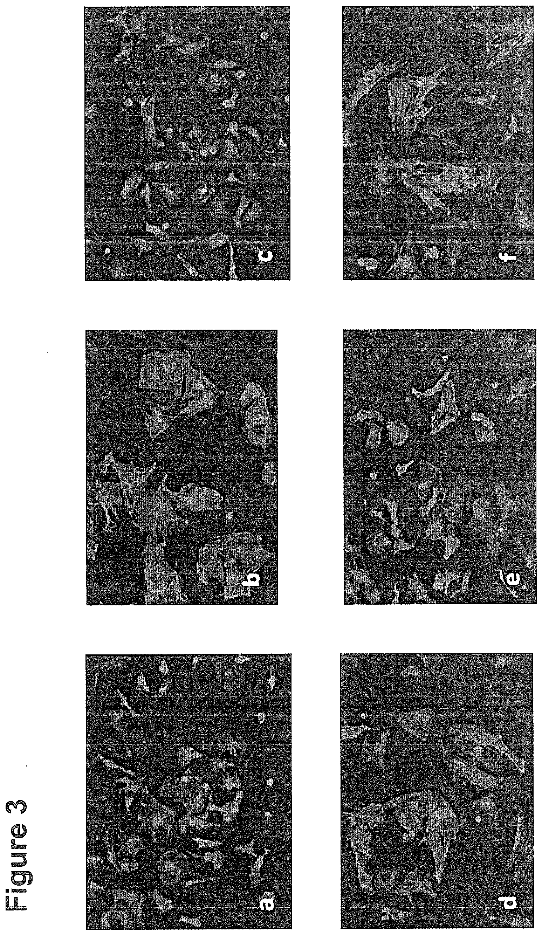

**Figure 3** shows, as a control experiment, the morphology of cardiomyocytes co-cultivated with HEK293 cells: a) serum-free without HEK293 cells; b) full medium (10% FCS) without HEK293 cells; c) serum-free with HEK293 cells (low density); d) full medium (10% FCS) with HEK293 cells (low density); e) serum-free with HEK293 cells (high density); f) full medium (10% FCS) with HEK293 cells (high density).

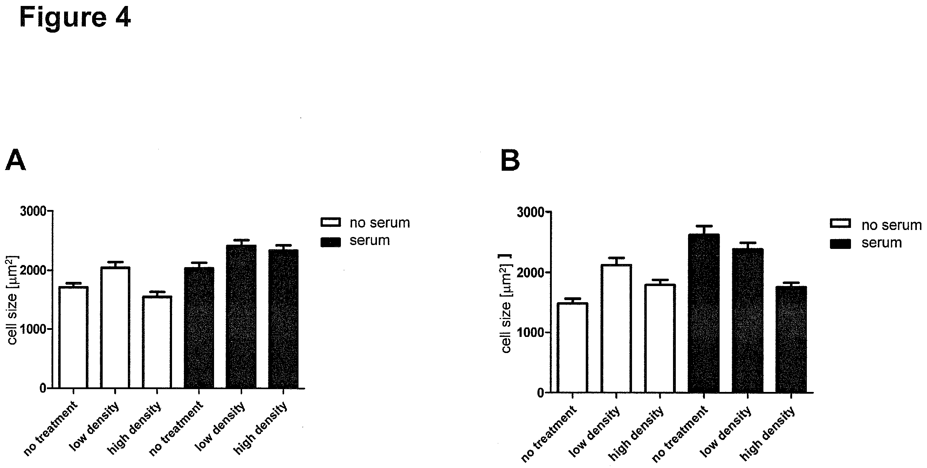

**Figure 4** shows the cell size of cardiomyocytes in co-culture with A) HEK293 cells and B) colon-26 tumor cells.

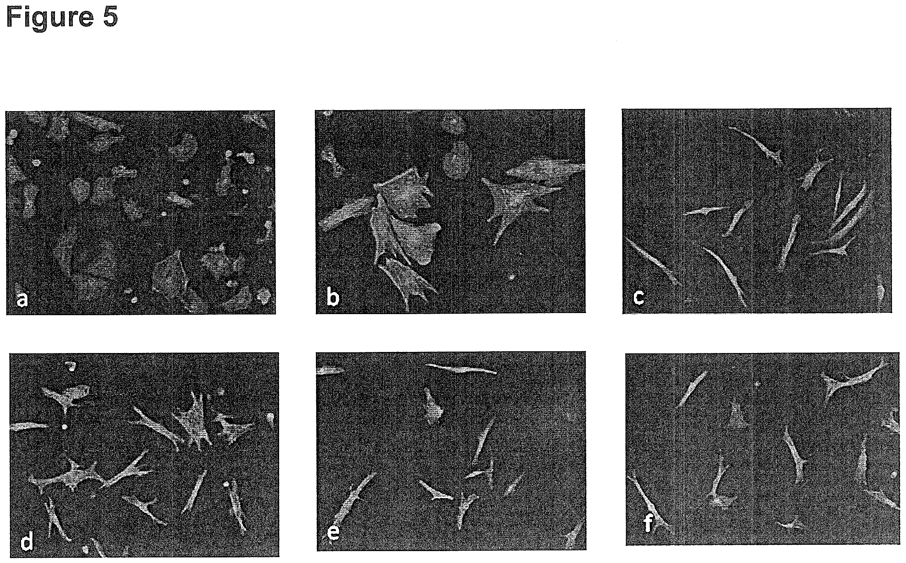

**Figure 5** shows the morphology of cardiomyocytes after treatment with conditioned medium of colon-26 tumor cells: a) cultivated in serum-free medium; b) cultivated in full medium; c) cultivated in conditioned medium (1:1 dilution with serum-free medium = low dose); d) cultivated in conditioned medium (1:1 dilution with full medium = low dose); e) cultivated in conditioned medium (2:1 dilution with serum-free medium = high dose); f) cultivated in conditioned medium (2:1 dilution with full medium = high dose).

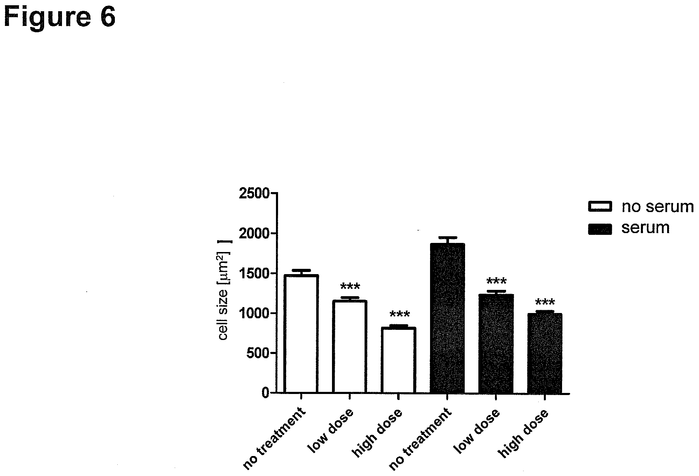

**Figure 6** shows the cell size of cardiomyocytes after treatment with conditioned medium of colon-26 tumor cells with or without serum (10% FCS). Significance: *p < 0,05; **p < 0,01; ***p < 0,001, ns = not significant.

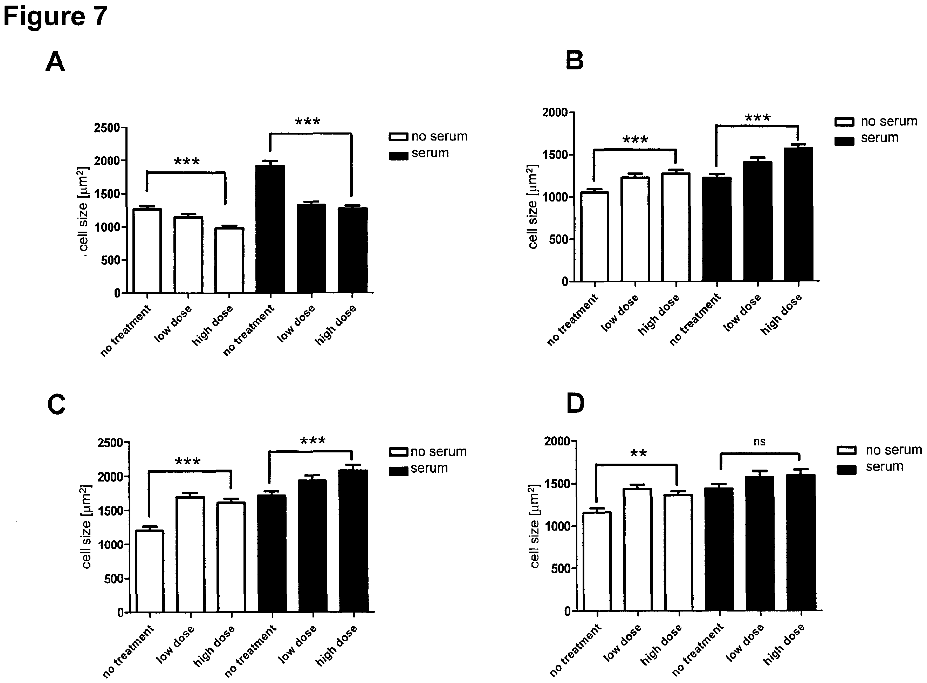

**Figure 7** shows the cell size after treatment with conditioned medium of human pancreatic tumor cell lines in different dilutions measured after 48 hours of incubation: A) BxPC-3; B) AsPC-1; C) Capan-1; D) Capan-2. Low dose = 1:1 dilution of conditioned medium, high dose = 2:1 dilution of conditioned medium. Significance: *p < 0,05; **p < 0,01; ***p < 0,001, ns = not significant.

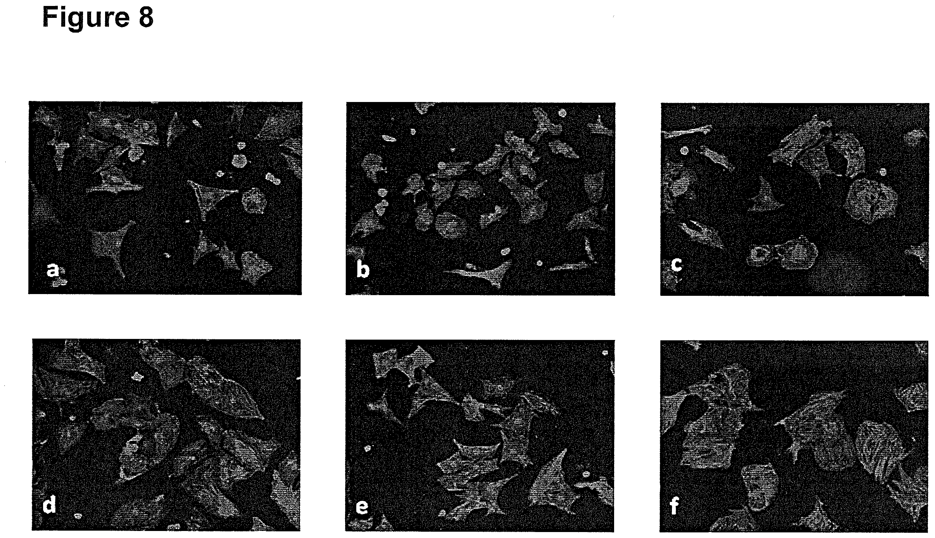

**Figure 8** shows the morphology of cardiomyocytes after treatment with conditioned medium of AsPC-1 cells: a) cultivated in serum-free medium; b) cultivated in full medium; c) cultivated in conditioned medium (1:1 dilution with serum-free medium = low dose); d) cultivated in conditioned medium (1:1 dilution with full medium = low dose); e) cultivated in conditioned medium (2:1 dilution with serum-free medium = high dose); f) cultivated in conditioned medium (2:1 dilution with full medium = high dose).

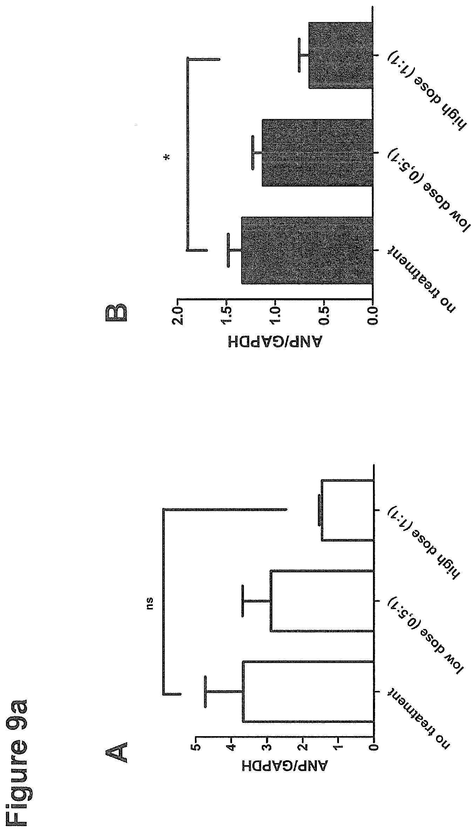

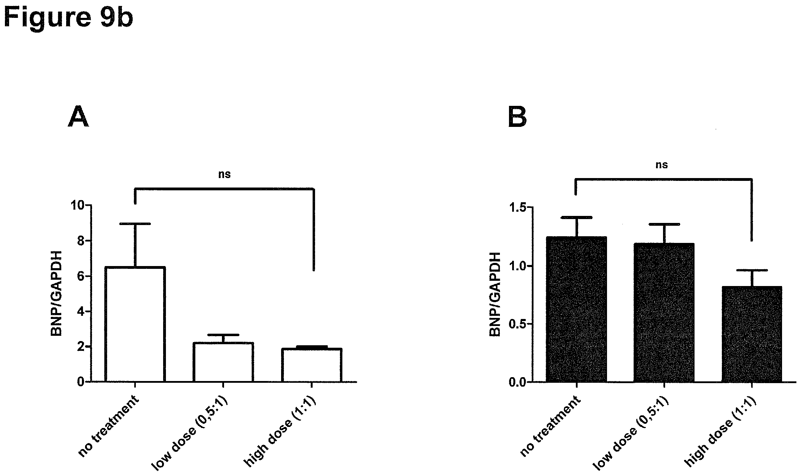

**Figure 9a** shows ANP expression in cardiomyocytes normalized to GAPDH expression measured via quantitative real-time PCR after 24 hour incubation with conditioned medium of colon-26 tumor cells; 0,5:1 dilution (low dose) and 1:1 dilution (high dose) under serum-free(A) and serum-enriched (B) conditions. N=3. Significance: *p < 0,05; **p < 0,01; ***p < 0,001, ns= not significant. **Figure 9b** shows BNP expression in cardiomyocytes normalized to GAPDH expression measured via RT-PCR.

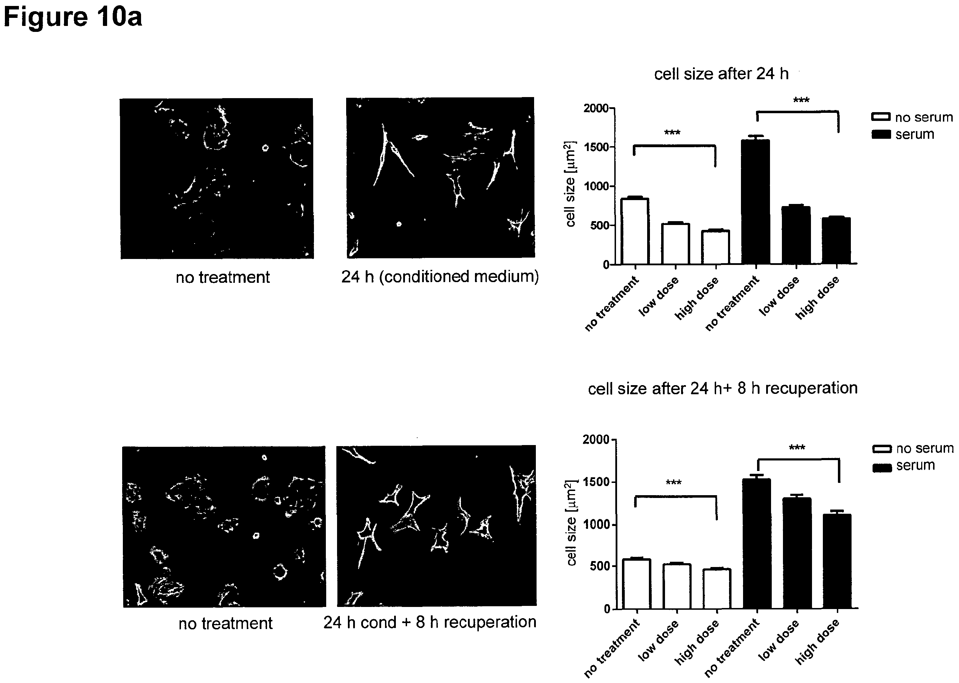

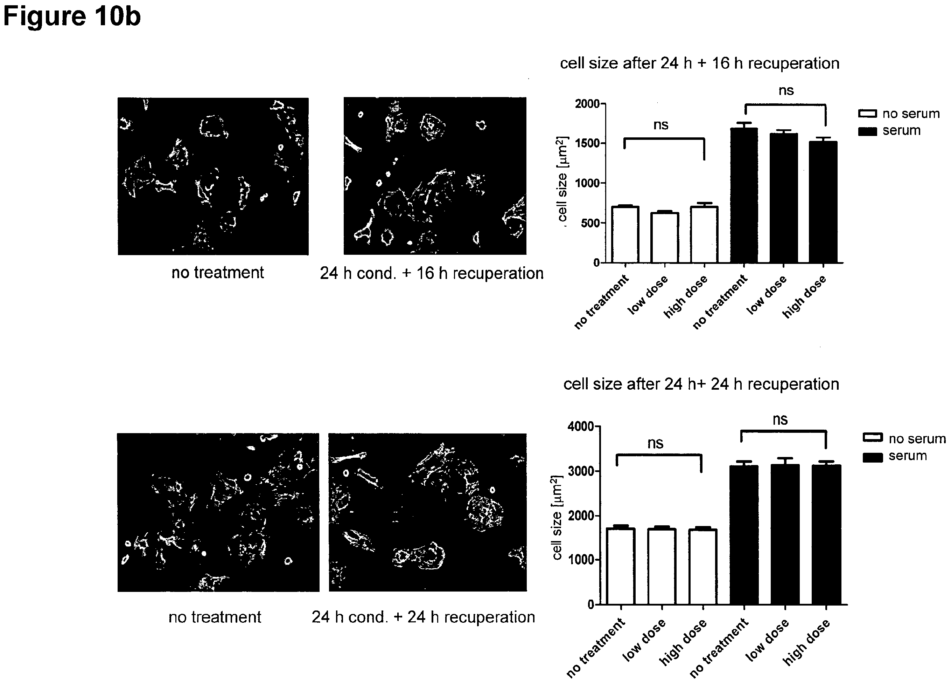

**Figures 10a** and **10b** show the morphology of cardiomyocytes after treatment with conditioned medium (colon-26) and different durations of recuperation.

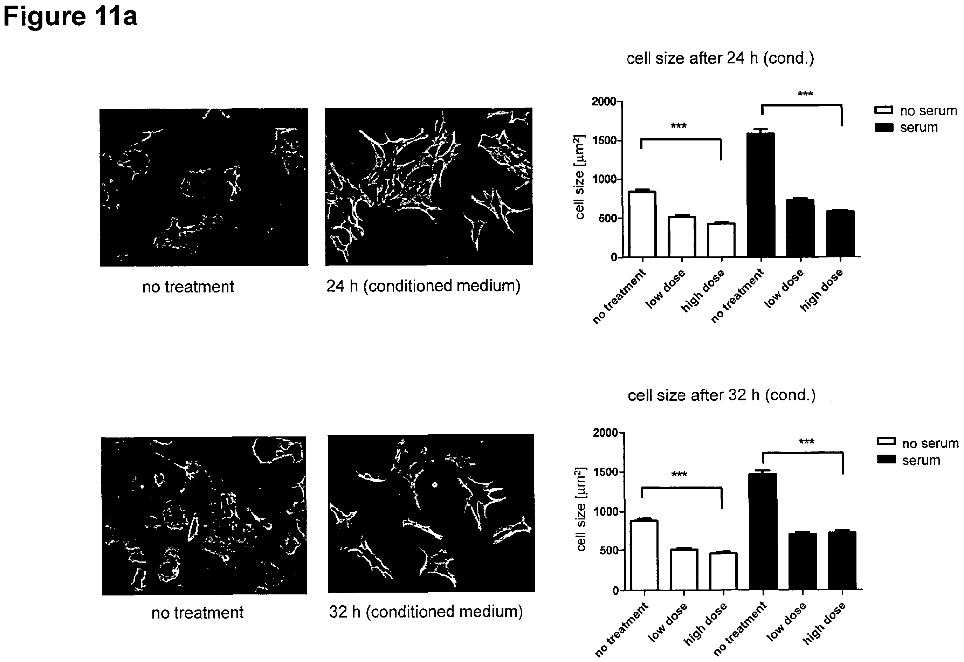

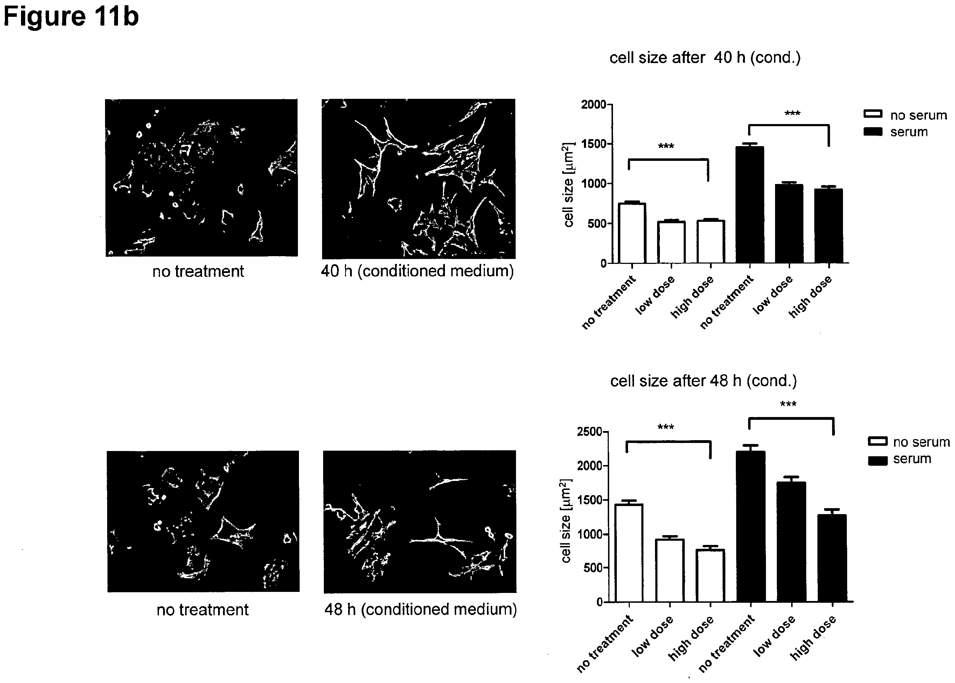

**Figures 11a** and **11b** show the morphology of cardiomyocytes after treatment with conditioned medium (colon-26) without a subsequent recuperation phase.

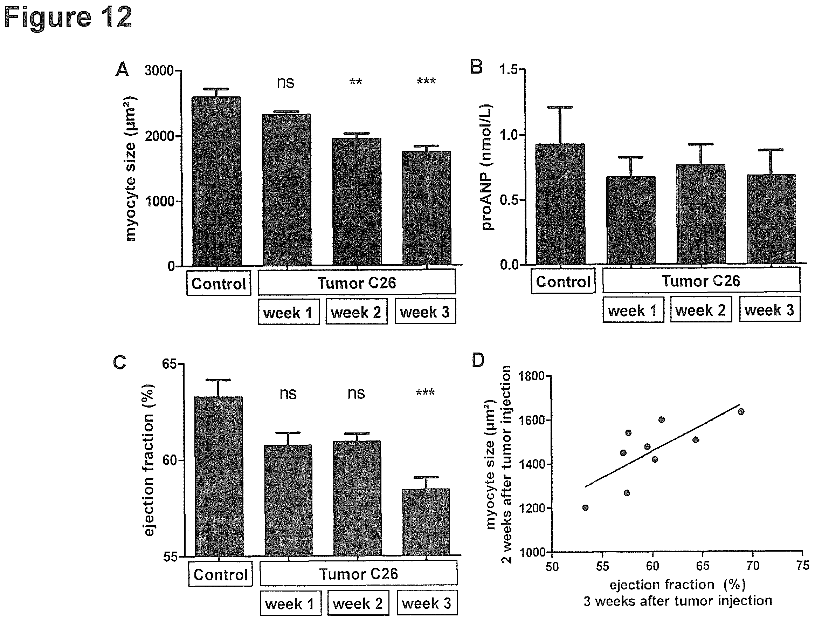

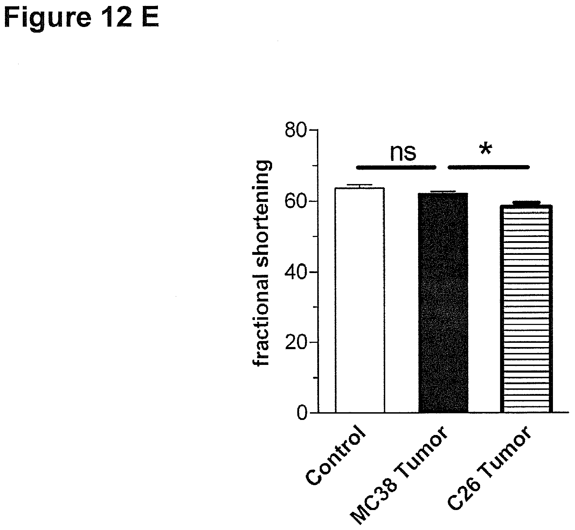

**Figure 12** (A) NRVMs were treated with serum of healthy control mice and mice at different time points after the onset of tumor growth upon injection of murine C26 colon cancer cells. Shown is the result of the serum-myocyte test. In parallel (B) proANP plasma levels and (C) the left ventricular ejection fraction were measured. (D) Correlation (r=0.76, p<0,01) between serum-myocyte test result (two weeks after C26 injection) with ejection fraction (three weeks after C26 injection). *p<0.05, **p<0.01, ***p<0.001, ns = not significant.
   (E) Comparison of the *in vivo* pump/contractile function of mice carrying a C26 tumor (poor or weak pump/contractile function) or of mice carrying a MC38 tumor (normal or good pump/contractile function).

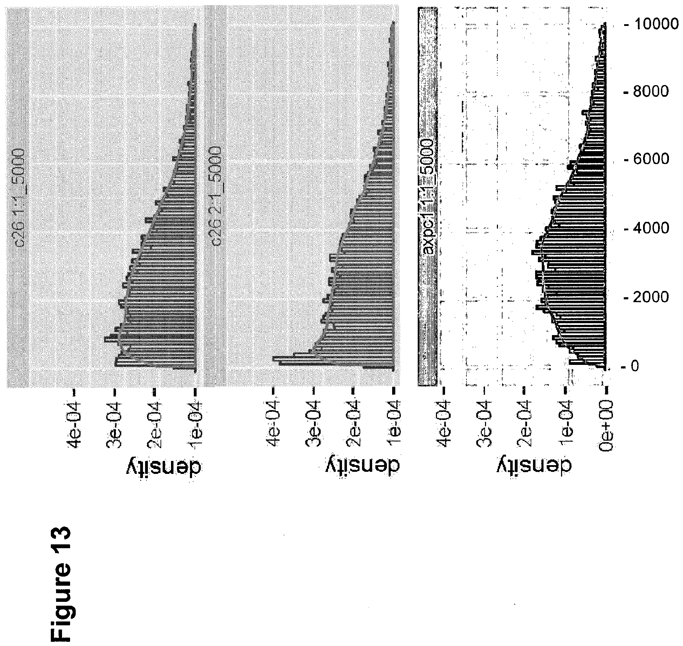

**Figure 13** shows the analysis of the effects of conditioned medium of tumor cells (c26 and AsPC-1) on cardiomyocytes using a 384-well high-throughput system (BD pathway system). The size of 40,000 cells per condition was automatically measured via camera-controlled and computed analysis. On the x-axis, different categories of cell size are indicated, whereas on the y-axis the quantity of cells in the particular category is indicated.

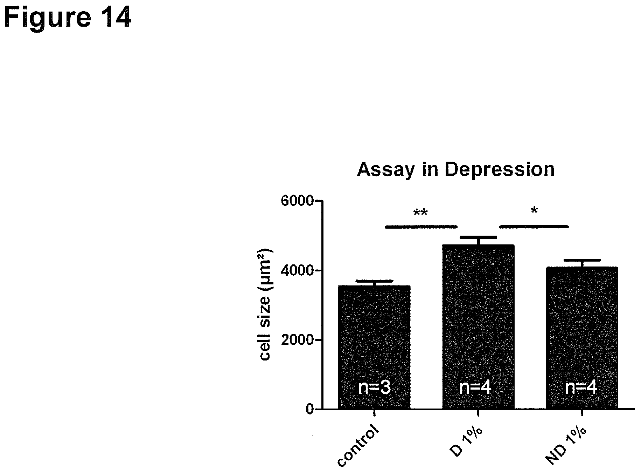

**Figure 14** shows the *in vitro* effects of blood serum derived from patients suffering from depression on cardiomyocyte size. Significance: **p<0,01; *p<0.05.

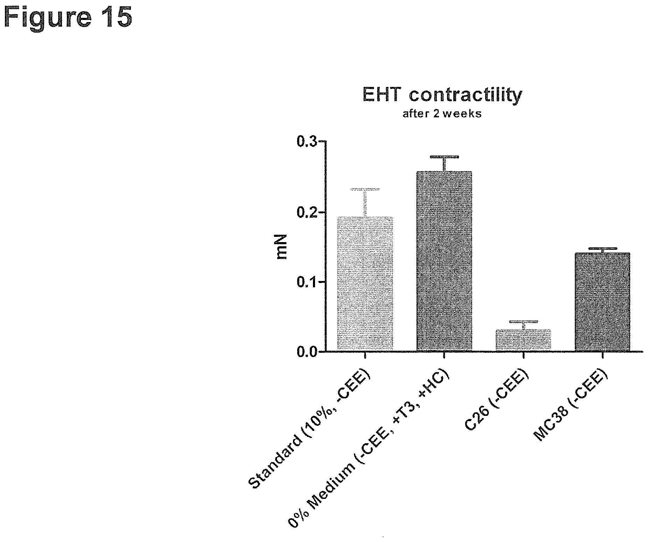

**Figure 15** shows measurement of the function of cardiomyocytes in an engineered heart tissue (EHT) model.

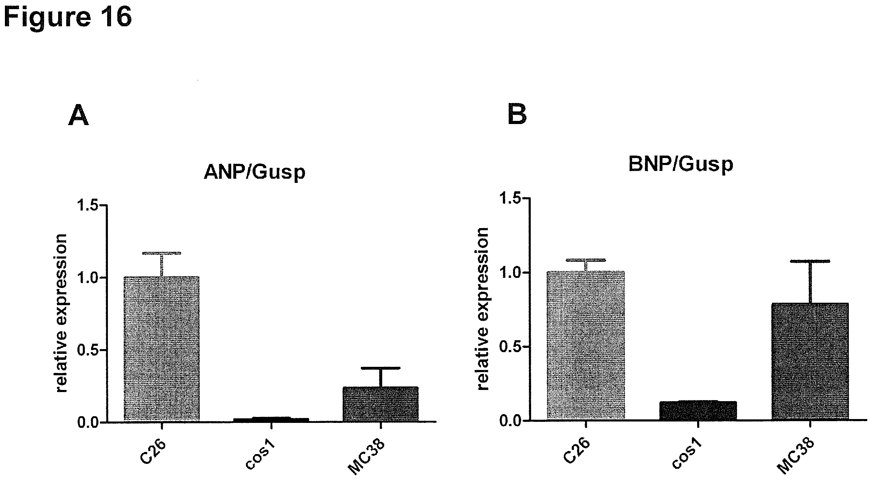

**Figure 16** shows gene expression in the engineered heart tissue (EHT) model.

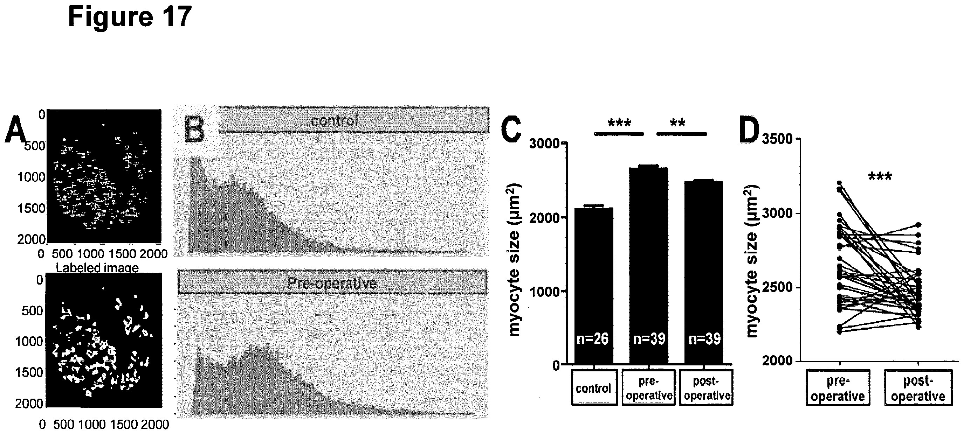

**Figure 17****.** (A) Representative images of NRVMs (double-positive for DAPI and alpha-actinin staining) in a single well of a 384 well plate captured with the BD pathway system in the wide-field mode (upper image). An algorithm was used to mark single cells with different colors to identify and quantify the size of single myocytes. The same measurements were also performed with the GE Healthcare InCell Analyser 2200 system. (B) Representative quantitative analyses of a single well, illustrating the distribution of all cells in the well. The y-axis indicates the percentage of the NRVMs, the x-axis the cell size (from left to right increasing sell size). (C) Quantitative group comparison of the serum-myocyte test result from the three patient groups. This quantitative analysis was performed with the InCell Analyser 2200 system. (D) Quantitative intra-individual comparison of the serum-myocyte test result obtained with plasma before surgery and after surgery. **p<0.01, ***p<0.001.

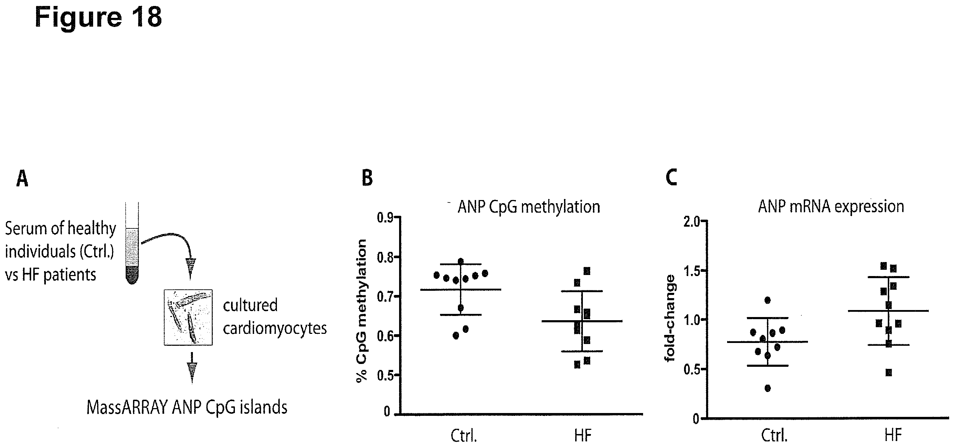

**Figure 18****.** Experiment "Indirect epigenetics". (A) Neonatal rat cardiomyocytes were cultured under addition of 1% serum of healthy individuals or HF patients (n=10 each) for 24 hours. (B) ANP CpG methylation was measured by MassARRAY analysis. (C) ANP mRNA was measured by real-time RT-PCR. *p<0.05.

### EXAMPLES

Reference is now made to the following examples, which are meant to illustrate the present invention and not to limit it:

### Materials & Methods

### Cell culture

Tumor cells were maintained in DMEM supplemented with 10% FCS, 2 mM 1-glutamine, and penicillin-streptomycin. Neonatal rat ventricular cardiomyocytes (NRVMs) were isolated from 1-2-d Sprague Dawley rats as previously described (Backs J. et al., J. Clin. Invest., 116(7):1853-64, 2006). After isolation, NRVMs were maintained in DMEM/199 medium (4:1) with 10% FCS, 2 mM 1-glutamine, and penicillin-streptomycin. Cells were cultivated in an incubator at 37°C with 5% CO₂.

The cell line "colon-26" (c26) originates from female Balb/c mice that were treated with N-nitroso-N-methylurethan. It is a carcinogenic epithelial tumor cell line. The cell line "MC38" is a cell line of a murine colon carcinoma. The cell line "HEK293" is a cell line derived from human embryonic kidney cells.

### Serum treatment

| | |
|---|---|
| **Control** | DMEM medium with or without 10% FCS ("full" or "starvation" medium, respectively) |
| **Low-Dose/ 1:1 dilution** | Conditioned medium diluted 1:1 with starvation or full medium |
| **High-Dose/ 2:1 dilution** | Conditioned medium diluted 2:1 with starvation or full medium |

### Co-culture experiments

Initially c26 tumor cells were co-cultivated with cardiomyocytes for 48 hours separated by a permeable membrane that allows exchange of secreted factors. High density (10⁴ tumor cells/well) and low density (10³ tumor cells/well) exposure to cardiomyocytes was performed.

### Histology

After fixation of the cells, immunofluorescence staining was performed with antibodies against sarcomeric alpha actinin (Sigma) as a cardiomyocyte marker and with DAPI (4',6-diamidino-2-phenylindole), a dye binding AT-rich DNA regions used for nuclear staining. All cells were captured at a magnification of x20. The size of cardiomyocytes was measured manually using appropriate imaging software (here: Olympus soft imaging software analySIS).

### 384-well high-throughput system

A BD pathway 855 automated microscope used for high-content assays and screens and appropriate robotics were used for the assay. Primary cardiomyocytes were automatically plated using a MultiDropDispenser (approximately 5000 per well, 8 wells per condition). 20,000 to 80.000 cells per condition were automatically analyzed after fluorescence staining with Hoechst, phalloidon and an anti-a-actinin antibody using Cellprofiler, an open-source software. Surface area of cardiomyocytes was measured to asses trophic effects of sera. Additionally, cell cycle analysis based an Hoechst staining was performed to detect apoptotic cells.

### Results

The inventors showed that not only in a co-culture model (Figure 1), but also via conditioned medium of cultivated tumor cells (Figures 5 to 8) both cardiomyocyte atrophy and hypertrophy can be induced in an isolated *in vitro* assay depending on the tumor cell line.

The inventors further demonstrated reproducibility (Figure 2) and specificity (Figure 3 to 4 and 7 to 8) of this assay. Moreover, they showed regulation of gene expression (Figure 9a and 9b) dependent on the serum used.

The inventors additionally showed reversibility of the effect of serum on cardiomyocytes via different periods of recuperation from serum exposure (Figure 10) as compared to constant exposure to conditioned medium (Figure 11) indicating atrophy as the mechanism underlying these conditions.

The inventors translated these findings to the blood circulation proving that serum drawn from mice diluted in culture medium can also induce similar effects (Figure 12).

### In vitro atrophy correlates with a reduced ejection fraction

Mice carrying a subcutaneous colon tumor (colon-26) develop a marked decrease in left ventricular ejection fraction three weeks after injection of the tumor in comparison to control mice (see Figure 12C).

The inventors compared the ejection fraction of the tumor bearing mice measured via echocardiography with the cell size measured in the *in vitro* assay according to the present invention. Nine animals (six c26 tumor-bearing mice and three control mice) were tested. The blood was drawn three weeks after injection of the tumor simultaneously with echocardiographic examination. Serum was diluted in culture medium (1%). Neonatal rat ventricular cardiomyocytes treated with the serum of the tumor-bearing mice showed reduced cell size, which correlated with reduced pump function (Figure 12D).

### High-throughput analysis

Data of an analysis of different conditioned media using a 384-well high-throughput system is shown in Figure 13. A left shift of the cell size distribution indicates atrophy (c26 medium on cardiomyocytes in 2 different dilutions 1:1 and 2:1, 5000 cells per well, 8 wells per condition: "c26 1:1_5000" and "c26 2:1_5000"), whereas a right shift as seen in "AsPC-1 1:1_5000" indicates a hypertrophic response of the cardiomyocytes due to exposure to conditioned medium of the pancreas tumor cell line AsPC-1. These data show that the method of the present invention can be upscaled and automated, so that multiple samples can be measured with high reliability. After further optimization of this high-throughput assay, patients' sera can be tested and then compared or correlated with the results of echocardiographic and standard laboratory data (e.g. NT-proBNP).

### Analysis of blood sera derived from patients with depressive disorders or colon carcinoma

The effects of blood sera of patients suffering from colon carcinoma on cardiomyocytes were examined (see Figure 17). The assay shows that incubation with serum of patients with colon carcinoma (performed in cell culture medium containing 10% FCS) resulted in NRVM hypertrophy as indicated by an increase in cell size. Medium with addition of serum of healthy patients was used as control sample.

Regulation of cardiomyocyte size *in vitro* could also be shown for blood serum derived from patients with depressive disorders (see Figure 15). Upon treatment with serum of these patients NRVMs showed a hypertrophic response. For this experiment the effect of 1% serum of 4 patients with depressive disorders (D) and 4 patients without depressive disorders (ND) in culture medium containing 10% FCS was tested on NRVMs. Medium without addition of patient serum, but with 10% FCS was used as control sample.

### Discussion

The inventors could show that conditioned medium of cultivated tumor cells can induce both cardiomyocyte atrophy and hypertrophy depending on the tumor cell line in an isolated *in vitro* assay. These data imply that there is a direct communication between tumor cells and cardiomyocytes via secreted factors. Immunological reactions that are supposed to play a major role in tumor cachexia do not seem to participate in this effect, as the inventors did not use any inflammatory cells in their approach. The inventors could show the *in vitro* effect on cardiomyocytes with human tumor cell lines as well as by using blood serum from tumor bearing mice or blood serum from cancer patients. The inventors treated neonatal rat ventricular cardiomyocytes (NRVM) with conditioned medium of a murine colon tumor cell line "colon-26" resulting in significant atrophy of the cardiomyocytes in comparison to cardiomyocytes treated with conditioned medium of the murine colon tumor mc-38. Based on this effect, systematic and automated high-throughput analyses can be performed. Cardiomyocytes can be treated with patients' sera and their effects, e.g. on cardiomyocyte size, can be measured. The morphometric changes of the cardiomyocytes (size, form, apoptosis and many more) can be used as an indicator to identify patients at risk of developing a cardiovascular/cardiac disease (e.g. atrophy or hypertrophy) and even as an indicator for the development of cachexia in tumor patients. An individual risk profile determined via the method of the present invention might be indicative for coronary artery disease, myocardial infarction, cardiomyopathies like dilatative or hypertrophic cardiomyopathy, cardiac involvement in certain systemic diseases and many more.

### Further results

Furthermore, the inventors confirmed their *in vitro* findings in a further *in vitro* but more physiological model, namely engineered heart tissue (EHT). Here they are able to measure the function of the cardiomyocytes (CardioF) as a complex muscular body. EHTs treated with supernatant (for about 14 days) from the different (tumor) cell lines show marked differences in contractility (in mN). Not only do the inventors see impaired function compared to standard EHT medium treated EHTs but C26 supernatant treated cardiomyocytes have massively reduced contractile function (as mice *in vivo* have shown, see Figure 12E) in comparison to (control tumor cell line) MC38 supernatant treated cardiomyocytes. See Figure 15.

In the same model, the inventors could confirm their findings regarding CardioGE. Gene expression in this EHT model was significantly regulated. High markers of the so-called fetal gene program (CardioGE) normalized to Gusp - a standard house keeping gene in EHTs - correlated with impaired function of EHTs (CardioF). Cos 1 as another control cancer cell line (human renal cancer) did not show elevation of the fetal gene program. See Figure 16.

### Studies with patient samples

The inventors could further show that the serum-myocyte test translates to cancer patients. Before the inventors started to use blood samples from patients, they developed an automated system to measure myocyte size. First, the inventors used the BD Pathway bioimaging system (BD Biosciences). The same measurements were later also performed with the InCell Analyser 2200 system (GE Healthcare), leading to almost identical results (see Figure 17A und B). Per plasma sample seven measurements in different wells of a 384 well plate were performed. The average standard deviation in these measurements was 246 µm². The inventors then treated NRVMs with plasma from patients suffering from colon carcinoma and compared them intra-individually to plasma taken after tumor resection to see whether the tumor load affects the serum-myocyte test. The inventors also compared them to healthy subject plasma that was taken in the same way as from the colon patients. The inventors found significant changes in NRVM size by both comparisons (Figure 17C and D).

### Indirect Epigenetic Assay (CardioE)

As disclosed herein, the inventors found that the treatment of isolated primary cardiomyocytes of rats with patient's blood serum samples leads to characteristic changes of cardiomyocyte size and morphology. The inventors can further show that this observation leads to an approach to indirectly measure epigenetic alterations that are mediated by metabolic features in the circulating blood. In a respective experiment, cultured neonatal rat ventricular myocytes (NRVMs) were treated with serum of patients with HF (n=10) versus serum of healthy individuals (n=10) for 24 hours and DNA CpG methylation changes (by MassARRAY) of the ANP gene were measured. Although the sample size was relatively small, significant changes were found towards hypomethylation. Since gene expression tends to be inversely correlated with promoter methylation, ANP mRNA levels were measured by qRT-PCR and, indeed, an increase in expression was found (see Figure 18).

The observation described above provides first evidence that the approach (according to the invention) allows one to indirectly detect epigenetic alterations in cardiomyocytes without the need to take myocardial biopsies but by simply adding patient blood serum to cultured cardiomyocytes.

This "Indirect Epigenetic Assay" (CardioE) can be performed with NRVMs but also with iPS-derived cardiomyocytes (iPSCs). The use of iPSCs enables one to use human cardiomyocytes, bringing the advantages (i) to be closer to the clinical application, (ii) to have no contaminations with non-cardiomyocytes, and (iii) to use the same techniques as used for the cost-effective analysis with the *Illumina Infinium 450 BeadChip* (which is compatible with human cells only).

## Claims

1. *In vitro* method for assessing the risk of developing a cardiovascular condition in a patient, said method comprising the steps:
- exposing a first group of cardiomyocytes to a sample derived from said patient;
- measuring at least one property of said cardiomyocytes;
- comparing said at least one property with the same at least one property measured in a second group of said cardiomyocytes, wherein said second group of said cardiomyocytes has been exposed to a control sample;
wherein a difference in said at least one property between said first group and said second group indicates an increased risk of developing said cardiovascular condition,
wherein said sample derived from said patient is blood serum or blood plasma, and said patient is selected from the group of tumor patients, patients with tumor-induced cachexia and patients suffering from depressive or other mood disorders, wherein said at least one property is selected from the group comprising cell size, cell morphology, rate of apoptosis, expression of one or more genes or reporter constructs, presence of one or more nucleic acid molecules, epigenetic profile, biochemical parameters and cell function.

2. *In vitro* method according to claim 1, wherein said cardiomyocytes are isolated from a non-human animal or said cardiomyocytes are derived from human or non-human induced pluripotent stem cells (iPSC).

3. *In vitro* method according to any of claims 1 to 2, wherein said cardiovascular condition is selected from the group comprising cardiac atrophy, cardiac hypertrophy, heart failure and hereditary or acquired cardiomyopathies.

4. *In vitro* method according to any of claims 1 to 3, wherein said control sample is a sample derived from a healthy individual or a mixture of samples derived from at least 3, preferably at least 5, more preferably at least 10 healthy individuals.

5. *In vitro* method according to any of claims 1 to 4, wherein said at least one property is cell size, wherein, preferably,
said difference is a decreased cell size in said first group of cardiomyocytes and said cardiovascular condition is cardiac atrophy, or
said difference is an increased cell size in said first group of cardiomyocytes and said cardiovascular condition is cardiac hypertrophy.

6. *In vitro* method according to any of claims 1 to 5, further comprising the step:
- comparing said at least one property with the same at least one property measured in a third group of said cardiomyocytes, wherein said third group of said cardiomyocytes has been exposed to a positive control sample for said cardiovascular condition.

7. *In vitro* method according to any of claims 1 to 6, wherein said cardiomyocytes are provided in a 3D-culture system.

8. *In vitro* method according to any of claims 1 to 7, wherein said step of measuring at least one property of said cardiomyocytes is performed with a plurality of properties of said cardiomyocytes, thereby establishing a cardiovascular risk profile of said patient.

9. Kit comprising
- a first container comprising cardiomyocytes, preferably cardiomyocytes derived from human or non-human induced pluripotent stem cells (iPSC);
- a second container comprising a control sample, which is blood serum or blood plasma, and which is a mixture of samples derived from at least 3, preferably at least 5, more preferably at least 10 healthy individuals; and
- optionally, at least one third container comprising a positive control sample for a cardiovascular condition.

10. Use of a kit according to claim 9 for assessing the risk of developing a cardiovascular condition in a patient and/or for establishing a cardiovascular risk profile of said patient, wherein said patient is selected from the group of tumor patients, patients with tumor-induced cachexia and patients suffering from depressive or other mood disorders.

## Patentansprüche

1. *In vitro* Verfahren zur Beurteilung des Risikos eines Patienten, eine Herz-Kreislauf-Erkrankung zu entwickeln, wobei das Verfahren die Schritte umfasst:
- Aussetzen einer ersten Gruppe von Kardiomyozyten einer Probe, die von dem Patienten stammt;
- Messen mindestens einer Eigenschaft der Kardiomyozyten;
- Vergleichen der mindestens einen Eigenschaft mit derselben mindestens einen Eigenschaft gemessen in einer zweiten Gruppe der Kardiomyozyten, wobei die zweite Gruppe der Kardiomyozyten einer Kontrollprobe ausgesetzt wurde;
wobei ein Unterscheid in der mindestens einen Eigenschaft zwischen der ersten Gruppe und der zweiten Gruppe anzeigt, dass ein erhöhtes Risiko besteht, die Herz-Kreislauf-Erkrankung zu entwickeln,
wobei die Probe, die von dem Patienten stammt, Blutserum oder Blutplasma ist, und der Patient ausgewählt ist aus der Gruppe von Tumorpatienten, Patienten mit Tumor-induzierter Kachexie und Patienten, die an depressiven Störungen oder anderen Stimmungsstörungen leiden,
wobei die mindestens eine Eigenschaft ausgewählt ist aus der Gruppe umfassend Zellgröße, Zellmorphologie, Apoptoserate, Expression von einem oder mehreren Genen oder Reporterkonstrukten, Anwesenheit von einem oder mehreren Nukleinsäuremolekülen, epigenetisches Profil, biochemische Parameter und Zellfunktion.

2. Das *in vitro* Verfahren gemäß Anspruch 1, wobei die Kardiomyozyten aus einem nicht-humanen Tier isoliert wurden oder die Kardiomyozyten von humanen oder nicht-humanen induzierten pluripotenten Stammzellen (iPSC) stammen.

3. Das *in vitro* Verfahren gemäß einem der Ansprüche 1 bis 2, wobei die Herz-Kreislauf-Erkrankung ausgewählt ist aus der Gruppe umfassend Herzatrophie, Herzhypertrophie, Herzversagen und angeborene oder erworbene Kardiomyopathien.

4. Das *in vitro* Verfahren gemäß einem der Ansprüche 1 bis 3, wobei die Kontrollprobe eine Probe ist, die von einem gesunden Individuum stammt, oder eine Mischung von Proben ist, die von mindestens 3, bevorzugt mindestens 5, weiter bevorzugt mindestens 10 gesunden Individuen stammen.

5. Das *in vitro* Verfahren gemäß einem der Ansprüche 1 bis 4, wobei die mindestens eine Eigenschaft die Zellgröße ist, wobei, bevorzugt,
der Unterschied eine verringerte Zellgröße in der ersten Gruppe der Kardiomyozyten ist und die Herz-Kreislauf-Erkrankung Herzatrophie ist, oder
der Unterschied eine vergrößerte Zellgröße in der ersten Gruppe der Kardiomyozyten ist und die Herz-Kreislauf-Erkrankung Herzhypertrophie ist.

6. Das *in vitro* Verfahren gemäß einem der Ansprüche 1 bis 5, weiterhin umfassend den Schritt:
- Vergleichen der mindestens einen Eigenschaft mit derselben mindestens einen Eigenschaft gemessen in einer dritten Gruppe der Kardiomyozyten, wobei die dritte Gruppe der Kardiomyozyten einer positiven Kontrollprobe für die Herz-Kreislauf-Erkrankung ausgesetzt wurde.

7. Das *in vitro* Verfahren gemäß einem der Ansprüche 1 bis 6, wobei die Kardiomyozyten in einem 3D-Kultursystem zur Verfügung gestellt werden.

8. Das *in vitro* Verfahren gemäß einem der Ansprüche 1 bis 7, wobei der Schritt des Messens der mindestens einen Eigenschaft der Kardiomyozyten mit einer Vielzahl von Eigenschaften der Kardiomyozyten durchgeführt wird, wobei ein Risikoprofil des Patienten für Herz-Kreislauf-Erkrankung etabliert wird.

9. Kit, umfassend
- einen ersten Behälter, umfassend Kardiomyozyten, bevorzugt Kardiomyozyten, die von humanen oder nicht-humanen induzierten pluripotenten Stammzellen (iPSC) stammen;
- einen zweiten Behälter, umfassend eine Kontrollprobe, welche Blutserum oder Blutplasma ist und welche eine Mischung von Proben ist, die von mindestens 3, bevorzugt mindestens 5, weiter bevorzugt mindestens 10 gesunden Individuen stammen; und
- optional, mindestens ein dritter Behälter umfassend eine positive Kontrollprobe für eine Herz-Kreislauf-Erkrankung.

10. Verwendung eines Kits gemäß Anspruch 9 zur Beurteilung des Risikos eines Patienten, eine Herz-Kreislauf-Erkrankung zu entwickeln, und/oder für das Etablieren eines Risikoprofils des Patienten für Herz-Kreislauf-Erkrankungen,
wobei der Patient ausgewählt ist aus der Gruppe von Tumorpatienten, Patienten mit Tumor-induzierter Kachexie und Patienten, die an depressiven Störungen oder anderen Stimmungsstörungen leiden.

## Revendications

1. Procédé *in vitro* d'estimation du risque de développer une affection cardiovasculaire chez un patient, ledit procédé comprenant les étapes de/d' :
- exposition d'un premier groupe de cardiomyocytes à un échantillon dérivé dudit patient ;
- mesure d'au moins une propriété desdits cardiomyocytes ;
- comparaison de ladite au moins une propriété à la même au moins une propriété mesurée dans un second groupe desdits cardiomyocytes, dans lequel ledit second groupe desdits cardiomyocytes a été exposé à un échantillon témoin ;
dans lequel une différence dans ladite au moins une propriété entre ledit premier groupe et ledit second groupe indique un risque augmenté de développer ladite affection cardiovasculaire,
dans lequel ledit échantillon dérivé dudit patient est du sérum sanguin ou du plasma sanguin, et ledit patient est choisi dans le groupe de patients atteints d'une tumeur, de patients atteints de cachexie induite par une tumeur et de patients souffrant de troubles dépressifs et autres de l'humeur, dans lequel ladite au moins une propriété est choisie dans le groupe comprenant la taille des cellules, la morphologie des cellules, le taux d'apoptose, l'expression d'un(e) ou de plusieurs gènes ou de constructions rapporteurs, la présence d'une ou de plusieurs molécules d'acide nucléique, le profil épigénétique, des paramètres biochimiques et la fonction cellulaire.

2. Procédé *in vitro* selon la revendication 1, dans lequel lesdits cardiomyocytes sont isolés à partir d'un animal non humain ou lesdits cardiomyocytes sont dérivés de cellules souches pluripotentes induites (CSPi) humaines ou non humaines.

3. Procédé *in vitro* selon l'une quelconque des revendications 1 ou 2, dans lequel ladite affection cardiovasculaire est choisie dans le groupe comprenant l'atrophie cardiaque, l'hypertrophie cardiaque, l'insuffisance cardiaque et les cardiomyopathies héréditaires ou acquises.

4. Procédé *in vitro* selon l'une quelconque des revendications 1 à 3, dans lequel ledit échantillon témoin est un échantillon dérivé d'un individu en bonne santé ou d'un mélange d'échantillons dérivés d'au moins 3, de préférence d'au moins 5, de façon davantage préférée d'au moins 10 individus en bonne santé.

5. Procédé *in vitro* selon l'une quelconque des revendications 1 à 4, dans lequel ladite au moins une propriété est la taille des cellules, dans lequel, de préférence,
ladite différence est une taille des cellules diminuée dans ledit premier groupe de cardiomyocytes et ladite affection cardiovasculaire est l'atrophie cardiaque, ou
ladite différence est une taille des cellules augmentée dans ledit premier groupe de cardiomyocytes et ladite affection cardiovasculaire est l'hypertrophie cardiaque.

6. Procédé *in vitro* selon l'une quelconque des revendications 1 à 5, comprenant en outre l'étape de :
- comparaison de ladite au moins une propriété à la même au moins une propriété mesurée dans un troisième groupe desdits cardiomyocytes, dans lequel ledit troisième groupe desdits cardiomyocytes a été exposé à un échantillon témoin positif pour ladite affection cardiovasculaire.

7. Procédé *in vitro* selon l'une quelconque des revendications 1 à 6, dans lequel lesdits cardiomyocytes sont fournis dans un système de culture tridimensionnelle.

8. Procédé *in vitro* selon l'une quelconque des revendications 1 à 7, dans lequel ladite étape de mesure d'au moins une propriété desdits cardiomyocytes est effectuée avec une pluralité de propriétés desdits cardiomyocytes, établissant de cette façon un profil de risque cardiovasculaire dudit patient.

9. Kit comprenant
- un premier récipient comprenant des cardiomyocytes, de préférence des cardiomyocytes dérivés de cellules souches pluripotentes induites (CSPi) humaines ou non humaines ;
- un deuxième récipient comprenant un échantillon témoin, qui est du sérum sanguin ou du plasma sanguin, et qui est un mélange d'échantillons dérivés d'au moins 3, de préférence d'au moins 5, de façon davantage préférée d'au moins 10 individus en bonne santé ; et
- éventuellement, au moins un troisième récipient comprenant un échantillon témoin positif pour une affection cardiovasculaire.

10. Utilisation d'un kit selon la revendication 9 pour estimer le risque de développer une affection cardiovasculaire chez un patient et/ou pour établir un profil de risque cardiovasculaire dudit patient, dans laquelle ledit patient est choisi dans le groupe de patients atteints d'une tumeur, de patients atteints de cachexie induite par une tumeur et de patients souffrant de troubles dépressifs ou autres de l'humeur.
